(19) Europäisches Patentamt
European Patent Office
Office européen des brevets

(11) **EP 4 057 053 A1**

(12) **EUROPEAN PATENT APPLICATION**

(43) Date of publication:
**14.09.2022 Bulletin 2022/37**

(21) Application number: **21305269.9**

(22) Date of filing: **08.03.2021**

(51) International Patent Classification (IPC):
**G02C 7/10** (2006.01)  **G02C 11/04** (2006.01)
**G02C 7/02** (2006.01)

(52) Cooperative Patent Classification (CPC):
**G02C 7/104; G02C 7/022; G02C 7/108;**
**G02C 11/04;** G02C 7/101; G02C 2202/24

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **ESSILOR INTERNATIONAL**
**94220 Charenton-Le-Pont (FR)**

(72) Inventors:
• **BARANTON, Konogan**
**94220 CHARENTON-LE-PONT (FR)**
• **BARRAU, Coralie**
**94220 CHARENTON-LE-PONT (FR)**
• **GUILLOT, Matthieu**
**94220 CHARENTON-LE-PONT (FR)**
• **VILLETTE, Thierry**
**94220 CHARENTON-LE-PONT (FR)**

(74) Representative: **Plasseraud IP**
**66, rue de la Chaussée d'Antin**
**75440 Paris Cedex 09 (FR)**

(54) **OPHTHALMIC SET FOR MYOPIA PROGRESSION CONTROL**

(57)  An ophthalmic set for myopia progression control comprises first means suitable for varying an amount of light that has wavelength values comprised in a modulation spectral range and enters an eye (E) of a user. It further comprises control means configured for controlling the first means so that light with wavelength values in the modulation spectral range enters the user's eye with varied intensity during daytime. The first means may comprise light sources (1, 2) and/or spectral filtering means, in particular arranged so as to be fitted on the user's face during appropriate durations. Preferably, light with wavelength values comprised between 360 nm and 520 nm, or between 440 nm and 520 nm, is increased in the morning and reduced in the evening.

FIG. 1

**Description**

The invention relates to an ophthalmic set for myopia progression control.

In the present description, the phrases myopia progression control, myopia progression slowing-down and myopia progression reduction are used with equivalent meanings.

## -- BACKGROUND OF THE INVENTION --

Myopia progression has been established through many observations and well documented for several years, although its cause(s) is (are) still subject to doubts and research. Myopia progression is the fact that for one person, his/her myopia increases with time at a rate which almost did not exist before. Kids are the most concerned with myopia progression, and it is thus a major issue for humanity to address this problem. Artificial light, in particular that produced by screens or LEDs, is suspected as being a cause for myopia progression, but the actual biological phenomena and mechanisms which lead to myopia progression remain at hypothesis level.

Several principles and methods have already been proposed for myopia progression control, including those now mentioned.

One of these methods consists in adding to spectacle lenses or contact lenses used for correcting myopia microlenses that focus part of light at a distance in front of the retina, in addition to the light that is focused on the retina for allowing sharp vision to the wearer who is equipped with these spectacle or contact lenses.

Another method consists in adding to the spectacle lenses or contact lenses used for correcting myopia non-spherical microlenses, in particular aspherical microlenses, that produce light volumes at a distance in front of the retina, again in addition to the light that is focused on the retina for producing the sharp image.

Still another method consists in adding to the spectacle lenses or contact lenses used for correcting myopia diffusing elements which reduce the vision contrast of the wearer.

Still other methods implement progressive addition lenses which provide power addition to compensate for the lag of accommodation, or bifocal prismatic lenses for producing both the power addition and a prism effect.

All these methods are based on lenses that are designed for modifying wavefronts of the light that enters the eyes of the wearer, or modifying wavefronts of part of this light.

Further methods are based on other principles, such as administering atropine to the subject, or wearing rigid contact lenses during nights for modifying the shape of the cornea.

But all these methods turn out not to be efficient enough in many cases for myopia progression control, so that new solutions are still required or even combinations of solutions.

Studies have been performed recently that suggest a role of the choroid in myopia progression. A thinning of the choroid is associated with a long-term length increase of the eyeball, which leads to myopia increase. In addition, it has been recently observed that light that enters the eyes in the morning causes choroid thickening, whereas light received in the evening causes choroid thinning. This would be due to biological circadian cycles, either local in the eyes or central for the person or even both. The wavelength range from 440 nm (nanometer) to 520 nm, corresponding to blue-green colours, is the one implied in central circadian regulation (with melanopsin absorption peaking at 480 nm) and is suspected playing a role for myopia progression. Another spectral range, from 560 nm to 600 nm and corresponding to amber light, also seems to play a role with circadian regulation and myopia progression, but in a lesser extent compared to blue-green light. Still another spectral range, from 360 nm to 400 nm, mainly 380 nm to 400 nm, corresponding to UV-violet light, might also be of interest in myopia progression, with a local circadian regulation by neuropsin.

Starting from this situation, one object of the present invention consists in providing new means for allowing myopia progression control, which are more efficient than those known from prior art.

Another object of the invention is that such means are easy for the user, without requiring much attention from him.

## -- SUMMARY OF THE INVENTION --

For meeting at least one of these objects or others, a first aspect of the present invention proposes an ophthalmic set for myopia progression control, which comprises:

- first means suitable for varying an amount of light that has wavelength values comprised in a modulation spectral range, and that enters an eye of a user of the ophthalmic set; and

- control means configured for controlling the first means within a period from wake-up to bedtime for the user, so that light with wavelength values comprised in the modulation spectral range, enters the user's eye with higher intensity during a first duration compared to a second duration, first and second durations forming an intraday time-configuration which is to be reproduced each day the ophthalmic set is used.

According to a further feature of the invention, the modulation spectral range is comprised between 360 nm and 520 nm, and preferably between 440 nm to 520 nm.

Therefore, the invention introduces a daily modulation in the intensity of the light in the modulation spectral range. Thanks to such intensity modulation, the myopia progression can be controlled for the user, because it allows inhibiting or correcting the circadian mechanisms that lead to progressive myopia increase.

Preferably, the control means may be configured so that the first duration starts from wake-up of the user and the second duration ends with bedtime of this user. Put another way, the first duration is focused on the morning period, and the second duration is focused on the evening period. Indeed, the efficiency of the invention for myopia progression control is higher when the user receives more light having wavelength value within the modulation spectral range in the mornings than in the evenings.

In first embodiments of the invention, the first means may comprise at least one light source effective in the modulation spectral range. Then, the control means are configured for activating light emission by the first means selectively during a light-supplementation period having a duration comprised between 15 minutes and 4 hours, preferably comprised between 30 minutes and 2 hours, from the wake-up of the user. Thus, such first embodiments of the invention strengthen the intensity as perceived by the user, of light with wavelength values within the modulation spectral range in the morning period.

For such first embodiments, the ophthalmic set may further comprise a frame to be worn by the user on his/her face, this frame supporting the at least one light source in a manner such that, when worn by the user, at least part of the light produced by the so-called at least one light source enters the user's eye.

Alternatively, the ophthalmic set may further comprise spectacles to be worn by the user, with two spectacle lenses accommodated in a spectacle frame. Then, the spectacles may have one of the following arrangements:

- light sources are accommodated in the spectacle frame close to a peripheral edge of each spectacle lens, and each spectacle lens is provided with light deflecting or diffusing elements suitable for directing at least part of the light produced by each light source into one of the user's eyes;

- light sources are accommodated in the spectacle frame or in temples thereof, and arranged so that light produced by the light sources reaches the spectacles lenses, and each spectacle lens is provided with a holographic diffusing layer adapted for directing at least part of the light into one of the user's eyes;

- light sources are accommodated in the spectacle frame or in temples thereof, and arranged so that light produced by the light sources reaches the spectacles lenses, and each spectacle lens is provided with an holographic layer that has a microlens pattern and is adapted for directing at least part of this light into one of the user's eyes;

- light sources are accommodated in the spectacle frame or in temples thereof, and arranged so that light produced by the light sources reaches the spectacles lenses, and each spectacle lens is provided with microlenses at a surface of this spectacle lens, or embedded within the spectacle lens or within a film which covers the spectacle lens; or

- light sources are comprised of at least one optical brightener dye or fluorescent compound distributed in or on the spectacle lenses, and UV sources are accommodated in the spectacle frame or in temples thereof and arranged so that UV radiation produced by these UV sources reaches the spectacles lenses.

For those of these arrangements that implement microlenses either at a surface of the spectacle lens, or embedded within the spectacle lens, or within a film which covers the spectacle lens, the microlenses may be non-spherical. They may be of refractive type, including unifocal, bifocal or aspherical, or of diffractive type, including pi-Fresnel type.

Advantageously for such embodiments, the control means may be configured for controlling an emission intensity of the at least one light source in a way such that a visual acuity loss or a vision contrast for the user remains matching a target value as the emission intensity varies.

In second embodiments of the invention, the first means may comprise spectral filtering means which have an average transmission value assessed over the modulation spectral range that is equal to or less than 50%, preferably less than 30%, and the control means are configured to cause the first means to be effective on light that enters the user's eye selectively during a light-dimming period ending with the bedtime of the user and having a duration comprised between 1 hour and 6 hours, preferably comprised between 2 hours and 4 hours.

Thus, such second embodiments of the invention strengthen the intensity decrease as perceived by the user, for light with wavelength values within the modulation spectral range, in the evening period.

Advantageously, the spectral filtering means may have another average transmission value assessed over another spectral range from 560 nm to 600 nm, that is less than 70%, preferably less than 50%. Indeed, this another spectral range 560 nm - 600 nm also participates in the myopia progression, although in a reduced extent compared to the ranges 360 nm - 520 nm and 440 nm - 520 nm. Therefore, the additional efficiency of the spectral filtering means between 560 nm and 600 nm increases the overall efficiency of the invention set for controlling myopia progression.

In possible embodiments, the spectral filtering means may be comprised of at least one absorbing dye which is distributed in or on an ophthalmic lens to be worn by the user.

Generally for the second invention embodiments, the spectral filtering means may be comprised in spectacles, or in a clip-on element to be affixed releasably to spectacles worn by the user, or in a patch to be affixed releasably to a spectacle lens worn by the user. Then, the control means may comprise alert means that are configured for informing the user to equip himself with the spectacles, clip-on element or patch when the light-dimming period starts. Alternatively, the control means may comprise light-measurement means which are adapted for measuring an intensity of ambient light. For such latter cases, the alert means may be coupled to the light-measurement means and configured for informing the user to equip himself with the spectacles, clip-on element or patch when the intensity of ambient light becomes less than a threshold. Advantageously, such threshold may equal 500 Lux, which corresponds to average light level at the beginning of the evening period, for both indoors and outdoors surroundings. The alert means may be provided through a smartphone application in simple embodiments of the invention.

In other second embodiments of the invention which are also possible, the spectral filtering means may be electrochromic means that are capable of switching between a blue-blocking state where the average transmission value assessed over the modulation spectral range is equal to or less than 50%, and a clear state where this average transmission value is higher than 50%. Then, the control means may be configured for switching the electrochromic means into the blue-blocking state when the light-dimming period starts. In a way similar as before, the control means may comprise the light-measurement means adapted for measuring the intensity of ambient light. Then the control means may be configured for switching the electrochromic means into the blue-blocking state when the intensity of ambient light becomes less than the threshold. This threshold may equal 500 Lux again.

Alternatively, for embodiments using spectral filtering means of electrochromic type, the control means may be configured for varying the electrochromic means progressively from the clear state to the blue-blocking state during the light-dimming period.

In still other second embodiments of the invention which are also possible, the spectral filtering means may comprise reverse photochromic means such that ambient radiation intensity above a transition threshold causes the reverse photochromic means to be in a clear state, and the ambient radiation intensity being below the transition threshold causes the reverse photochromic means to be in a blue-blocking state. For such operation, the transmission value of the reverse photochromic means assessed over the modulation spectral range is lower in the blue-blocking state compared to the clear state.

Third embodiments of the invention may combine any one of the first embodiments as recited above, with any one of the second embodiments. Thus, the first means comprise at least one light source which is effective in the modulation spectral range, and the control means are configured for activating light emission by this at least one light source as recited for the first embodiments. In addition, the first means also comprise spectral filtering means which have an average transmission value assessed over the modulation spectral range that is equal to or less than 50%, preferably less than 30%, and the control means are further configured to cause the spectral filtering means to be effective on light that enters the user's eye as recited for the second embodiments.

Generally for the invention, the control means may comprise an activity measurement unit that is configured for determining a wake-up time of the user and his bedtime. Then for such improvement of the invention, the control means may be configured for controlling the first means based on the wake-up time as determined by the activity measurement unit, and/or on bedtime as determined from at least one prior use of the ophthalmic set.

A second aspect of the invention proposes a process for maintaining vision comfort to a person, in particular to a child, when this process comprises providing this person with the ophthalmic set of the first invention aspect, and the person using the ophthalmic set in daily life.

These and other features of the invention will be now described with reference to the appended figures, which relate to preferred but not-limiting embodiments of the invention.


**-- BRIEF DESCRIPTION OF THE DRAWINGS --**


Figure 1 illustrates a first embodiment of the invention, using light sources.

Figure 2 illustrates second embodiments of the invention, also using light sources.

Figure 3 illustrates third embodiments of the invention.

Figure 4 shows transmission spectra of filters that may be used in the third embodiments of the invention.

For clarity sake, element sizes which appear in these figures do not correspond to actual dimensions or dimension ratios. Also, same reference numbers which are indicated in different ones of these figures denote identical elements of elements with identical function.

## -- DETAILED DESCRIPTION OF THE INVENTION --

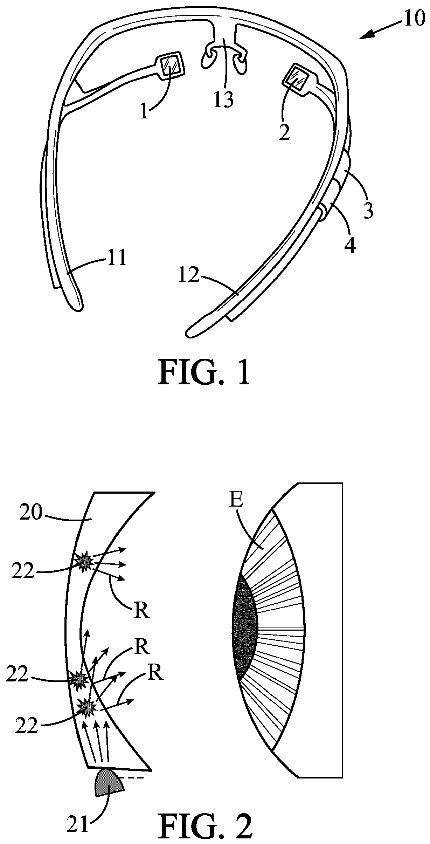

First and second embodiments of the invention now described with reference to Figures 1 and 2 implement light sources. These light sources are used for supplementing the user with light in the wavelength range of 360 nm - 520 nm, preferably 440 nm - 520 nm, and optionally also in the range 560 nm - 600 nm. They may be controlled using means that will be described later, and which are compatible with any embodiment based on light sources.

Turning to Figure 1, a frame 10 is designed for fitting the user's face, with temples 11 and 12 and nasal support 13. The frame 10 may be designed to be light, comfortable and for not hindering vision for the user. The frame 10 supports at least one light source, for example two light sources 1 and 2, so that these latter are located and oriented for directing light into the user's eyes without masking a significant part of his vision field. For example, the frame 10 may comprise two extensions suitable for maintaining each light source 1 and 2 at a distance from one the eyes in a lower part of the corresponding vision field. In particular, each light source may be thus maintained at 3 cm (centimetre) to 5 cm from the corresponding eye. Alternatively, the light sources 1 and 2 may be supported by the transverse beam of the frame 10 in front of the user's eyebrows. The sources 1 and 2 are selected for emitting light mainly in the wavelength range of 360 nm - 520 nm, preferably 440 nm - 520 nm, possibly with additional emission in the range 560 nm - 600 nm. LED technology may advantageously be used for the light sources 1 and 2, because of low energy consumption, small volume and light-weight features. Batteries 4 may be accommodated in the frame 10, for example in the temples 11 and 12, and also control means 3. The batteries 4 are suitable for supplying energy to the light sources 1 and 2, and the control means 3 are designed for activating or switching off the light sources 1 and 2 according to a schedule which will be described later.

Turning now to Figure 2, a spectacle frame (not represented) supports a spectacle lens 20 in front of each one of the user's eye, in a common way. Each lens 20 may have a spherical power value suitable for correcting a myopia of the wearer, but the invention may also be used for a person devoid of myopia in order to avoid that such myopia appears and increases later for this person. For such latter case, the lens 20 is devoid of spherical power, as commonly referred to as plano lens. The spectacle frame incorporates at least one light source 21 which is located close to the edge of one of the lenses 20, and arranged to transmit light within this lens from its edge to a center part of the lens. Then, the lens 20 is provided with light-deflecting means suitable to re-direct the light rays emitted by the light source 21 towards the user's eye. E denotes the user's eye and R denotes light rays as re-directed towards the eye E by the light-deflecting means. The light source 21 to be used in such second embodiments may be similar to those of the first embodiment, with similar battery arrangement and control means. Suitable light-deflecting means may be light-diffusing elements 22 which are distributed within the lens material. Alternatively, such light-diffusing elements 22 may be distributed across the front or back face of the lens 20. For example, they may be formed by small patterns engraved in the lens face, or in a transparent film or coating which is supported by the lens face. Possibly, such light-diffusing elements 22 may be replaced by a holographic diffusing layer which is supported by the front or back face of the lens. Other possibilities consist in providing the lens 20 with microlenses suitable for deflecting light from the light source 21 toward the user's eye. Such microlenses may be non-spherical. They may also be of refractive type, including unifocal, bifocal or aspherical, or of diffractive type, including pi-Fresnel type. In various embodiments, such microlenses may be located either at a surface of the lens 20, or embedded within the lens 20, or within a film which covers the lens 20.

Still another possibility is to provide the front or back face of the lens with a holographic layer having a microlens pattern.

Alternatively, a UV source may be used instead of the light source 21 which was effective in the wavelength range 360 nm - 520 nm or 440 nm - 520 nm, and the lens 20 is now provided with at least one optical brightener dye or fluorescent compound suitable for absorbing the UV radiation as produced by the UV source. This optical brightener dye or fluorescent compound re-emits light in the wavelength range 360 nm - 520 nm or 440 nm - 520 nm. Part of this light as re-emitted by the optical brightener dye is oriented towards the corresponding user's eye E so that it enters into it. Similar arrangements are provided for both of the user's eyes.

The control means 3 are configured for activating the light source(s) or UV source(s) during a predetermined period in daytime. In this way, the user's eyes can receive higher amounts of light having wavelength values between 360 nm and 520 nm, or between 440 nm and 520 nm, selectively during this predetermined period. In preferred embodiments of the invention, the control means are configured so that the activation period of the sources starts at the wake-up time of the user and continues for a duration which may be up to 4 hours, preferably at least 15 minutes, most preferably of between 30 minutes and 2 hours. Such light-supplementation period promotes choroid thickening and thereby limits myopia progression for the user. Possibly, the light dose for the supplementation may be varied depending on the user, in particular depending on a thickness value measured for his choroid.

Several designs for the control means may be used alternatively.

According to first possible designs of the control means, they may comprise a photodetector suitable for measuring an intensity of ambient light as existing in the surroundings of the user. Such photodetector may be based on a photodiode and accommodated in the frame 10 of the first embodiment or the spectacle frame of the second embodiment. It identifies the wake-up time of the user through daylight appearance in the user's surroundings, and then the control means activate continuously the light sources or UV sources during the predetermined light-supplementation period.

According to other possible designs of the control means, the photodetector may be replaced with an inertial measurement unit also accommodated in the frame 10 or spectacle frame, and suitable for sensing the first displacements of the frame in the morning. The time of such first displacements is identified as the user's wake-up time, and the operation of the control means is the same as before.

According to still other possible designs of the control means, they may be located in the frame 10 or spectacle frame and incorporate a wireless communication unit for being connected to an external personal assistant device such as a smartphone. Then, the starting time for activating the light sources or UV sources may be determined by a dedicated application hosted by the smartphone. It may be based on the first time in the day the user makes use of his smartphone, for example.

Preferably but optionally, an intensity of the supplemental light that is delivered by the light sources or the UV sources via the optical brightener dye may be adjusted during the light-supplementation period for maintaining a visual acuity level or contrast level for the user. Indeed, the intensity of the supplemental light may cause a reduction in the contrast effective for the user when viewing at anything in his surroundings. For avoiding that such contrast reduction could reduce vision comfort substantially, it is possible to adjust the intensity of the supplemental light depending on the intensity of ambient light. For example, the control means may adjust the intensity of the supplemental light so that its ratio to the ambient light intensity matches a target value. The light-diffusing elements distributed across the spectacle lenses may also cause a reduction in the visual acuity. So the intensity of the supplemental light may be alternatively adjusted by the control means so that the visual acuity level for the user remains higher than a predetermined threshold. Preferably, when light-diffusing elements 22 are thus implemented in the lenses, they may be preferably located in a peripheral area of each lens 20 so that the central area of this lens still provides high vision acuity. In this way, it is possible to combine good visual acuity through the center areas of the spectacle lenses with an efficiency of the peripheral areas of the lenses for slowing-down myopia progression.

The other invention embodiments described below are intended to reduce the amount of light with wavelength values between 360 nm - 520 nm, or between 440 nm and 520 nm, that enters the user's eyes during a determined period called light-dimming period. Such light-dimming period limits choroid thinning and thereby slows down myopia progression for the user. Although the following embodiments are described with spectacle type equipment, the Man skilled in the art will be able to adapt them to contact lenses without difficulty. Such embodiments based on contact lenses are therefore also within the scope of the invention as defined by the claims. For instance, it is possible to set the filtering area in a ring of each contact lens with a pupil diameter greater than 3 mm. It permits to filter light only when the user's pupil is larger than 3 mm, i.e. not outside or during day.

For clarity sake, the present description continues with the filtered spectral range of 440 nm - 520 nm, but it applies similarly by replacing this range of 440 nm - 520 nm with the extended one of 360 nm - 520 nm.

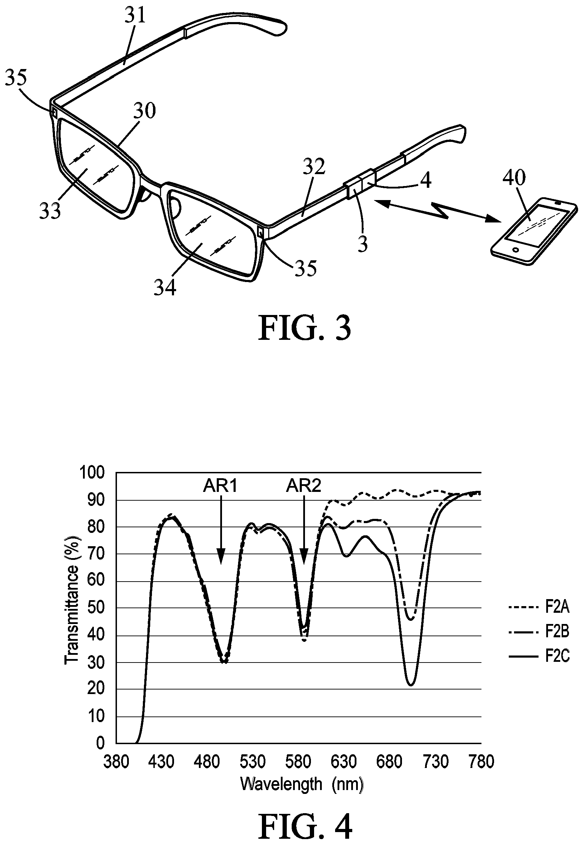

Figure 3 shows a spectacle equipment with spectacle frame 30, temples 31 and 32, and spectacle lenses 33 and 34. Each lens 33, 34 is provided with spectral filtering means which reduce the amount of light with wavelength values between 440 nm and 520 nm that this lens transmits to one of the user's eye. Such spectral filtering means may be comprised of one or more light-absorbing dyes which are incorporated in the material of the lenses 33 and 34. Alternatively, such dyes may be contained in films which are covering at least one face of each lens 33, 34. These dyes are selected for providing each lens with an average transmission value of less than 50%, preferably less than 30%, over the spectral range from 440 nm to 520 nm. Indeed, 440 nm - 520 nm has been identified as the most important range for the choroid thickness variations related to circadian rhythm. Since the other spectral range from 560 nm to 600 nm has been also observed with effect on the choroid thickness variations, the dyes may be advantageously further selected for being also light-absorbing between 560 nm and 600 nm, preferably in a way to provide each lens with another average transmission value of less than 70%, preferably less than 50%, over the spectral range 560 nm - 600 nm. In addition to strengthening the efficiency for myopia progression control as produced by the light reduction in the first range 440 nm - 520 nm, the additional light reduction in the second range 560 nm - 600 nm improves the colour rendering for the user through the lenses 33 and 34, when compared to light reduction only in 440 nm - 520 nm. The Man skilled in the art knows how to select light-absorbing dyes from spectral features thereof as provided by chemical suppliers, and how to adjust dye concentrations within the material of the lenses 33, 34 for obtaining desired transmission values. Appropriate dyes for obtaining the spectral features recited above are provided in WO 2019/238648. The diagram of Figure 4 shows three transmission spectra that are appropriate for the lenses 33 and 34 for implementing of the invention. These spectra are labelled F2A, F2B and F2C, respectively. The horizontal axis of the diagram identifies the wavelength values in nanometers (nm), and the vertical axis identifies the spectral transmission values in %. Arrow AR1 points a first transmission minimum in the range 440 nm - 520 nm, and arrow AR2 points that in the other range 560 nm - 600 nm.

The average transmission value over the range 440 nm - 520 nm is assessed as a mean value of spectral transmission values that relate respectively to wavelength values comprised between 440 nm and 520 nm, with uniform weighting factors. Put another way, this average transmission value is calculated according to the following equation:

$$T(440 \text{ nm} - 520 \text{ nm}) = (1/80) \cdot \int_{440}^{520} T(\lambda) \cdot d\lambda$$

where the wavelength values $\lambda$ are expressed in nanometers, the summation interval is from 440 nm to 520 nm, and the spectral transmission values $T(\lambda)$ are expressed in %. Alternatively, the blue-green wavelength interval may be extended to 360 nm - 520 nm, so that the equation for the average transmission value can be also:

$$T(360 \text{ nm} - 520 \text{ nm}) = (1/160) \cdot \int_{360}^{520} T(\lambda) \cdot d\lambda$$

In preferred embodiments of the invention, at least one of T(440 nm - 520 nm) and T(360 nm - 520 nm) is less than 50%, preferably less than 30%. It is called average blue-green transmission value.

The average amber transmission value may be assessed, over the further spectral range from 560 nm to 600 nm and corresponding to amber light. Such average amber transmission value may be calculated according to:

$$T(560 \text{ nm} - 600 \text{ nm}) = (1/40) \cdot \int_{560}^{600} T(\lambda) \cdot d\lambda$$

In preferred embodiments of the invention, T(560 nm - 600 nm) is less than 70%, preferably less than 50%.

For implementing the invention, the filtering spectacles of Figure 3 may be provided to the user with alert means configured to inform him about the time to equip himself with the spectacles. In first possible embodiments, such alert means may be comprised of an application hosted in a personal assistant device of the user such as a smartphone 40. For example, such application may be designed for determining an expected bedtime of the user, for example based on times of last use sessions of the smartphone by the user in preceding days, and/or light switch-off times as detected by the smartphone light sensor in the preceding days. Then, the application may be configured to alert the user for equipping with the filtering spectacles for example 2 hours before the expected bedtime.

In possible embodiments, the control means may incorporate at least one ambient light sensor which may be accommodated in the spectacle frame 30, as indicated by reference 35. This light sensor performs measurements of the ambient light level as existing in the surroundings of the user and supplies the measurement results to a wireless communication unit also accommodated in the spectacle frame 30. Then the measurement results are retransmitted to the smartphone 40. For such operation, the smartphone 40 may be wireless connected to the communication unit of the spectacle frame 30. Then, the alert for the user to equip with the spectacles may be issued by the smartphone 40 once the ambient light becomes lower than a threshold, thereby indicating evening time. Such threshold may be set to 500 Lux, for example.

Similar operation may be obtained based on an activity measurement unit which is accommodated in the spectacle frame 30. Such activity measurement unit may comprise one or several of the following components: an inertial measurement unit suitable for movement detection, a photodetector suitable for sensing light variations, a clock, an acoustic sensor suitable for detecting sounds or speech existing in the surroundings of the user, etc. Then the activity measurement unit may be based on combined operations of these components. Activity detection signals as provided by such activity measurement unit are transmitted to the smartphone 40 for determining the expected bedtime from the data accumulated in the preceding days. An activity measurement unit which is accommodated in the smartphone may be used alternatively, and operatively coupled to the alert means.

Although these latter embodiments have been described referring to spectacles as represented in Figure 3, it is possible to provide similar embodiments that are based on clip-on elements or patches. In such cases, the filtering means are incorporated in a clip-on element or patch, and this latter has to be mounted on spectacles that the user wears permanently. Such spectacles may be suitable for permanently correcting an ametropia of the user, and the clip-on element is to be affixed on the spectacle frame selectively for the evening time so that light passes through both the spectacle lenses and the lenses of the clip-on element. Patches are to be adhered by the user onto the spectacle lenses for the evening time, and then removed when using the spectacles again in the morning of the next day.

Other possible embodiments may be based on electrochromic technology, to allow the user to wear the spectacles permanently. The spectacle lenses may be provided with dioptric power and/or astigmatism for compensating a user's ametropia. Electrochromic technology makes it possible to activate temporarily spectral filtering means, for example selectively during 2 hours before bedtime. To this end, the spectacle lenses 33 and 34 are provided with respective electrochromic devices across their whole optical areas. Electrochromic compounds are selected to provide a clear state with average blue-green transmission value that is higher than 50%, and a blue-blocking state with the average blue-green transmission value that is less than 50%. The spectacle frame 30 accommodates batteries 4 suitable for causing switching of the electrochromic devices from clear state to blue-blocking state, and also the reverse operation. The control means 3, also accommodated in the spectacle frame 30, are configured to trigger switching of the electrochromic devices from clear state to blue-blocking state in the evening, when the light-dimming period starts. Such embodiments based on electrochromic technology may be combined with ambient light sensor and/or activity measurement unit and/or smartphone application as before. One advantage of combining the electrochromic technology with an ambient light sensor is that the spectacles can be autonomous for producing myopia progression control, without requiring a smartphone.

According to an improvement of the electrochromic-based embodiments, the transition from clear state to blue-blocking state may be soft, with a progressive evolution of the light transmission between both states. This avoids discomfort caused by sudden switch. Indeed, soft transition may not be detected by the spectacle wearer, since it may be partially compensated for by his pupil variations.

Still other possible embodiments may be based on reverse-photochromic technology. Reverse-photochromic technology is based on chemical compounds that are absorbing in the visible range when ambient radiation is low-level, i.e. below a transition threshold, but become non-absorbing when the level of the ambient radiation is above the transition threshold. For most reverse-photochromic compounds, the spectral range which controls absorption transition in the visible range, belongs to UV domain. It is thus possible to select reverse-photochromic compounds that are sensitive to solar UV radiation as existing in outdoor surroundings, but not present in indoors surroundings or under artificial lighting. The operation of such embodiments of the invention is very simple, because of being based only on the fact that UV radiation suitable for maintaining the clear state is lacking in the evenings, whatever the indoor or outdoor surroundings of the user at that time. With a proper time constant of UV activation, it is also possible to maintain a clear state for intermittent indoor period during the day, and an absorption state in the evening.

Secondary aspects of the invention embodiments described above may be varied easily, in particular regarding the exact implementation of the control means. One will understand that the invention produces daily variations of the light intensity that is received in each eye in the wavelength range 360 nm - 520 nm, but it can be controlled in numerous ways, some of them requiring action from the user and others being automatic or autonomous.

**Claims**

1. An ophthalmic set for myopia progression control, comprising:

   - first means suitable for varying an amount of light that has wavelength values comprised in a modulation spectral range, and that enters an eye (E) of a user of the ophthalmic set; and
   - control means configured for controlling the first means within a period from wake-up to bedtime for the user, so that light with wavelength values comprised in the modulation spectral range enters the user's eye (E) with higher intensity during a first duration compared to a second duration, first and second durations forming an intraday time-configuration which is to be reproduced each day the ophthalmic set is used,

   the modulation spectral range being comprised between 360 nm and 520 nm.

2. The ophthalmic set of claim 1, wherein the modulation spectral range is comprised between 440 nm and 520 nm.

3. The ophthalmic set of claim 1 or 2, wherein the control means are configured so that the first duration starts from wake-up of the user and the second duration ends with bedtime of said user.

4. The ophthalmic set of any one of the preceding claims, wherein the first means comprise at least one light source (1, 2; 21) effective in the modulation spectral range, and the control means are configured for activating light emission by the first means selectively during a light-supplementation period having a duration comprised between 15 minutes and 4 hours, preferably comprised between 30 minutes and 2 hours, from the wake-up of the user.

5. The ophthalmic set of claim 4, further comprising a frame (10) to be worn by the user on the user's face, said frame supporting the at least one light source (1, 2) in a manner such that, when worn by the user, at least part of the light produced by said at least one light source enters the user's eye (E).

6. The ophthalmic set of claim 4, further comprising spectacles to be worn by the user, with two spectacle lenses (20) accommodated in a spectacle frame, and wherein the spectacles have one of the following arrangements:

- light sources (21) are accommodated in the spectacle frame close to a peripheral edge of each spectacle lens (20), and each spectacle lens is provided with light deflecting or diffusing elements (22) suitable for directing at least part of the light produced by each light source into one of the user's eyes (E);
- light sources (21) are accommodated in the spectacle frame or in temples of said spectacle frame, and arranged so that light produced by said light sources reaches the spectacles lenses (20), and each spectacle lens is provided with a holographic diffusing layer adapted for directing at least part of said light into one of the user's eyes (E);
- light sources (21) are accommodated in the spectacle frame or in temples of said spectacle frame, and arranged so that light produced by said light sources reaches the spectacles lenses (20), and each spectacle lens is provided with an holographic layer that has a microlens pattern and is adapted for directing at least part of said light into one of the user's eyes (E);
- light sources (21) are accommodated in the spectacle frame or in temples of said spectacle frame, and arranged so that light produced by said light sources reaches the spectacles lenses (20), and each spectacle lens is provided with microlenses at a surface of said spectacle lens, or embedded within said spectacle lens or within a film which covers said spectacle lens; or
- light sources are comprised of at least one optical brightener dye or fluorescent compound distributed in or on the spectacle lenses (20), and UV sources are accommodated in the spectacle frame or in temples of said spectacle frame and arranged so that UV radiation produced by said UV sources reaches the spectacles lenses.

7. The ophthalmic set of claim 6, wherein the control means are configured for controlling an emission intensity of the at least one light source (21) in a way such that a visual acuity loss or a vision contrast for the user remains matching a target value as the emission intensity varies.

8. The ophthalmic set of any one of claims 1 to 3, wherein the first means comprise spectral filtering means which have an average transmission value assessed over the modulation spectral range that is equal to or less than 50%, preferably less than 30%, and the control means are configured to cause the first means to be effective on light that enters the user's eye (E) selectively during a light-dimming period ending with the bedtime of the user and having a duration comprised between 1 hour and 6 hours, preferably comprised between 2 hours and 4 hours.

9. The ophthalmic set of claim 8, wherein the spectral filtering means have another average transmission value assessed over another spectral range from 560 nm to 600 nm, that is less than 70%, preferably less than 50%.

10. The ophthalmic set of claim 8 or 9, wherein the spectral filtering means are comprised of at least one absorbing dye distributed in or on an ophthalmic lens (33, 34) to be worn by the user.

11. The ophthalmic set of any one of claims 8 to 10, wherein the spectral filtering means are comprised in spectacles, or in a clip-on element to be affixed releasably to spectacles worn by the user, or in a patch to be affixed releasably to a spectacle lens (33, 34) worn by the user, and
the control means comprise alert means configured for informing the user to equip himself with the spectacles, clip-on element or patch when the light-dimming period starts,
or the control means comprise light-measurement means (35) adapted for measuring an intensity of ambient light, and alert means coupled to the light-measurement means and configured for informing the user to equip himself with the spectacles, clip-on element or patch when the intensity of ambient light becomes less than a threshold.

12. The ophthalmic set of claim 8 or 9, wherein the spectral filtering means are electrochromic means capable of switching between a blue-blocking state where the average transmission value assessed over the modulation spectral range is equal to or less than 50%, and a clear state where said average transmission value is higher than 50%, and the control means are configured for switching the electrochromic means into the blue-blocking state when the light-dimming period starts,
or the control means comprise light-measurement means adapted for measuring an intensity of ambient light, and said control means are configured for switching the electrochromic means into the blue-blocking state when the intensity of ambient light becomes less than a threshold.

13. The ophthalmic set of claim 8 or 9, wherein the spectral filtering means are electrochromic means capable of varying between a blue-blocking state where the average transmission value assessed over the modulation spectral range is equal to or less than 50%, and a clear state where said average transmission value is higher than 50%, and the control means are configured for varying the electrochromic means progressively from the clear state to the blue-blocking state during the light-dimming period.

14. The ophthalmic set of claim 8 or 9, wherein the spectral filtering means comprise reverse photochromic means such that ambient radiation intensity above a transition threshold causes the reverse photochromic means to be in a clear state, and the ambient radiation intensity being below the transition threshold causes the reverse photochromic means to be in a blue-blocking state, with a transmission value of the reverse photochromic means assessed over the modulation spectral range that is lower in the blue-blocking state compared to the clear state.

15. The ophthalmic set of any one of claims 1 to 3, wherein the first means comprise at least one light source effective (1, 2; 21) in the modulation spectral range, and the control means are configured for activating light emission by said at least one light source so that the ophthalmic set meets any one of claims 4 to 7, and the first means further comprise spectral filtering means which have an average transmission value assessed over the modulation spectral range that is equal to or less than 50%, preferably less than 30%, and the control means are further configured to cause the spectral filtering means to be effective on light that enters the user's eye (E) so that the ophthalmic set also meets any one of claims 8 to 14.

16. The ophthalmic set of any one of the preceding claims, wherein the control means comprise an activity measurement unit that is configured for determining a wake-up time of the user and the bedtime of said user, and the control means are configured for controlling the first means based on said wake-up time as determined by the activity measurement unit, and/or on bedtime as determined from at least one prior use of the ophthalmic set.

17. Process for maintaining vision comfort to a person, in particular to a child, said process comprising providing said person with the ophthalmic set of any one of the preceding claims, and the person using said ophthalmic set in daily life.

FIG. 1

FIG. 2

FIG. 3

FIG. 4

Europäisches
Patentamt

European
Patent Office

Office européen
des brevets

# EUROPEAN SEARCH REPORT

Application Number

EP 21 30 5269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2019/204624 A1 (BARRAU CORALIE [FR] ET AL) 4 July 2019 (2019-07-04)<br>* Paragraphs 11-15, 24, 36, 40-47, 51-52, 76-79, 87, 88, 125-128, 169, 175, 213; figure 3 * | 1-9, 12-17<br>10,11 | INV.<br>G02C7/10<br>G02C11/04<br>G02C7/02 |
| X | WO 2020/025355 A1 (ESSILOR INT [FR]) 6 February 2020 (2020-02-06)<br>* paragraphs [0005] - [0009], [0030], [0086], [0024]; figure 4 * | 1,4-8, 12-14,17 | |
| X | US 2019/258086 A1 (BARRAU CORALIE [FR] ET AL) 22 August 2019 (2019-08-22)<br><br>* paragraphs [0007], [0018], [0033] - [0037], [0085] * | 1,3, 8-10, 12-17 | |
| X | US 2021/001145 A1 (MASON STEPHEN [AU]) 7 January 2021 (2021-01-07)<br>* paragraphs [0008] - [0013] * | 1,4,17 | |
| Y | CN 206 002 793 U (SHANGHAI YUDE COMM TECHNOLOGY CO LTD) 8 March 2017 (2017-03-08)<br>* paragraph [0030] * | 10,11 | TECHNICAL FIELDS SEARCHED (IPC)<br><br>G02C |
| Y<br>A | CN 209 248 184 U (ZHU YONGGANG) 13 August 2019 (2019-08-13)<br>* paragraph [0016] * | 10,11<br>12-14 | |

The present search report has been drawn up for all claims

| Place of search | Date of completion of the search | Examiner |
|---|---|---|
| Munich | 30 July 2021 | Albero Silvestre, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 5269

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2021

| Patent document cited in search report | | | Publication date | Patent family member(s) | | | Publication date |
|---|---|---|---|---|---|---|---|
| US 2019204624 | A1 | | 04-07-2019 | CN | 108885357 | A | 23-11-2018 |
| | | | | EP | 3232254 | A1 | 18-10-2017 |
| | | | | US | 2019204624 | A1 | 04-07-2019 |
| | | | | US | 2021157172 | A1 | 27-05-2021 |
| | | | | WO | 2017178430 | A1 | 19-10-2017 |
| WO 2020025355 | A1 | | 06-02-2020 | CN | 112513721 | A | 16-03-2021 |
| | | | | EP | 3830637 | A1 | 09-06-2021 |
| | | | | US | 2021231979 | A1 | 29-07-2021 |
| | | | | WO | 2020025355 | A1 | 06-02-2020 |
| US 2019258086 | A1 | | 22-08-2019 | CN | 108431677 | A | 21-08-2018 |
| | | | | EP | 3394665 | A1 | 31-10-2018 |
| | | | | JP | 2019502953 | A | 31-01-2019 |
| | | | | KR | 20180096636 | A | 29-08-2018 |
| | | | | US | 2019258086 | A1 | 22-08-2019 |
| | | | | WO | 2017108976 | A1 | 29-06-2017 |
| US 2021001145 | A1 | | 07-01-2021 | AU | 2019226631 | A1 | 29-10-2020 |
| | | | | CN | 111757768 | A | 09-10-2020 |
| | | | | EP | 3758797 | A1 | 06-01-2021 |
| | | | | JP | 2021515959 | A | 24-06-2021 |
| | | | | US | 2021001145 | A1 | 07-01-2021 |
| | | | | WO | 2019165507 | A1 | 06-09-2019 |
| | | | | WO | 2019165508 | A1 | 06-09-2019 |
| CN 206002793 | U | | 08-03-2017 | NONE | | | |
| CN 209248184 | U | | 13-08-2019 | NONE | | | |

EPO FORM P0459

For more details about this annex : see Official Journal of the European Patent Office, No. 12/82

**EP 4 057 053 A1**

REFERENCES CITED IN THE DESCRIPTION

**Patent documents cited in the description**

- WO 2019238648 A